# EUROPEAN PATENT APPLICATION

(11) **EP 4 759 231 A1**
(43) Date of publication of application: **17.06.2026**
(21) Application number: 25223149.3
(22) Date of filing: 12.12.2025
(51) Int. Cl.: A61B 5/145, A61B 5/1459, A61B 5/1473, B23K 26/20, H05K 5/06, B23K 20/02, B32B 15/04, A61B 5/1455

(54) **IMPLANTABLE OPTICAL SENSOR HAVING A SAPPHIRE WINDOW MICRO-SCALE DIFFUSION BONDED TO A TITANIUM HOUSING**

(30) Priority: 13.12.2024 US 202463733469 P
(71) Applicant: Greatbatch Ltd., Clarence, NY 14031 (US)
(72) Inventor: RUBINO, Robert S., Williamsville, 14221 (US); GANGULY, Adrish, Clarence, 14031 (US); KOCH, Melissa, Spencerport, 14559 (US); ZHOU, Boyang, Buffalo, 14215 (US)
(74) Representative: Mathys & Squire

(57) **Abstract**

An implantable optical sensor having a sapphire window as an optically transparent ceramic micro-scale diffusion bonded to a titanium washer to form a sapphire window/titanium washer assembly is described. The titanium washer is then welded to the inner surface of a device housing in alignment with a housing opening. The micro-scale diffusion bond is formed using an electromagnetic beam emanating from a Gaussian-Bessel laser and has a thickness that is greater than 1 µm, and preferably ≥ 4 µm, with a weld width that is preferably ≥ 45 µm. Importantly, the diffusion bond is spaced from and intermediate undisturbed portions of the optically transparent sapphire and the opaque conductive titanium. A power source connected to an optical sensor aligned with the sapphire window is housed inside the device housing.

## Description

### CROSS-REFERENCE TO RELATED APPLICATION

This application claims priority to U.S. provisional application Serial No. 63/733,469, filed on December 13, 2024.

### FIELD OF THE INVENTION

The present invention generally relates to the field of micro-scale diffusion bonding of dissimilar materials. More particularly, the present invention relates to micro-scale diffusion bonding an optically transparent insulating ceramic to an opaque metallic conducting material such as a conductive metal. Single crystalline Al₂O₃ (sapphire) is a preferred optically transparent ceramic and titanium is a preferred conductive metal. A Gaussian-Bessel pulsed laser beam may be used to create the micro-scale diffusion bond between the dissimilar materials. The diffusion bonded optically transparent sapphire to a titanium housing is particularly advantageous in an optical sensor.

### BACKGROUND OF THE INVENTION

The formation of a diffusion bond between bulk materials with dissimilar properties has potential applications in many industries. For example, in the semiconductor industry, the ability to diffusion bond an insulating material to a conducting material is highly desired. Further, forming a diffusion bond between FDA approved biocompatible materials such as optically transparent, insulating, single crystalline Al₂O₃ (common name: sapphire) and conductive titanium or titanium alloys has multiple applications including formation of the housing for an implantable or a non-implantable medical device or a photonic device, and in jewelry manufacturing, among a host of other applications.

For example, U.S. Patent No. 10,124,559 to Sandlin et al. describes the creation of a nano-scale diffusion bond having a thickness that is less than 1,000 nm or 1 µm between sapphire as an optically transparent ceramic and titanium as an opaque conductive metal. The diffusion bond is created using a laser pulse in the 5 µm to 15 µm range, a laser spot size of around 10 µm, an overlap in the range of 1-10%, a frequency in the range of 1 kHz to 80 kHz, and a pulse energy of 1 µJ to 5 µJ with the electromagnetic beam being moved relative to the titanium-sapphire bulk materials at a rate of 5 mm/s to 600 mm/s. The laser produces a UV 355 nm-IR 1064 wavelength, has an average power of 2.1 mW-100 mW, and a repetition rate (frequency of pulses) of about 1 kHz to create a uniform and continuous bond having a thickness less than 1,000 nm between the sapphire and titanium.

Interestingly, the Sandlin et al. patent relates that increasing the laser pulse energy to 2.5 µJ or more while keeping the other parameters discussed above constant resulted in a bond with visible cracking. However, with the laser pulse energy being less than 2.0 µJ, a bond did not form. Additionally, the Sandlin et al. patent discusses that other wavelengths across the entire UV, visible and infrared spectra can be used. For example, a laser with a wavelength of 532 nm, a pulse energy of 1 uJ, a spot size of 10 µm, a pulse frequency of 1 kHz, and a pulse overlap of 50% can be used to achieve the desired less than 1,000 nm diffusion bond.

While the creation of a diffusion bond having a thickness that is less than 1,000 nm is sufficient to connect the dissimilar sapphire and titanium bulk materials together, there is a need to produce a diffusion bond with increased strength and hermeticity. That is particularly the case when the diffusion bond between sapphire and titanium is intended for incorporation into the housing of an implantable or a non-implantable medical device or photonic device, and for inclusion of an optically transparent sapphire window in a titanium housing for an optical sensing device. These types of devices are intended to be implanted into a human or animal body for extended periods of time, which means that the diffusion bond must be robust enough to withstand the rigors of an implantable environment without cracking or developing imperfections that would render the implantable device compromised.

As will become apart to those skilled in the art after having read the detailed description in view of the appended drawings, the present invention accomplishes those goals.

### SUMMARY OF THE INVENTION

Aspects and embodiments of the present invention are defined in the appended claims.

In one embodiment of the present invention, a kinetically limited micro-scale diffusion bond having a thickness greater than about 1 µm, and preferably ≥ 4 µm connects dissimilar bulk materials together. The connected bulk materials include sapphire as an optically transparent insulating ceramic, titanium, or a titanium alloy as an opaque conductive metal, and a kinetically limited micro-scale diffusion bond connecting the two materials together. The optically transparent sapphire has properties that allow an electromagnetic (EM) beam, preferably a Gaussian-Bessel pulse EM energy beam of a select wavelength to pass through it without more than minimal energy absorption. The opaque conductive metal has properties that significantly absorb energy from the electromagnetic beam. The micro-scale diffusion bond is formed by the electromagnetic beam bonding the optically transparent sapphire to the opaque titanium. Moreover, the micro-scale diffusion bond has a diffusion bond penetration or thickness that is greater than 1 µm and is preferably ≥ 4 µm, and may have a diffusion bond width that ranges from about 1 µm to about 200 µm and is preferably ≥ 45 µm.

In yet another embodiment, a kinetically limited micro-scale diffusion bond having a thickness that is ≥ 4 µm connects dissimilar bulk materials together. In this embodiment, the bulk materials having the kinetically limited micro-scale diffusion bond include sapphire as an optically transparent ceramic, an undisturbed portion of the optically transparent sapphire, titanium, or a titanium alloy as an opaque conductive metal, an undisturbed portion of the opaque conductive metal, and an interfacial micro-scale diffusion bond connecting the optically transparent sapphire to the opaque conductive titanium. The interfacial micro-scale diffusion bond is spaced from and intermediate undisturbed portions of the sapphire and the titanium.

The optically transparent sapphire has properties that allow a Gaussian-Bessel electromagnetic beam of a select wavelength to pass through it without more than minimal energy absorption. The opaque titanium has properties that significantly absorb energy from the Gaussian-Bessel electromagnetic beam. The micro-scale diffusion bond is formed by the electromagnetic beam bonding the optically transparent sapphire to the opaque titanium or titanium alloy, and has a thickness that is greater than 1 µm, and more preferably that is ≥ 4 µm. Preferably, the diffusion bond also has a diffusion bond width that ranges from about 1 µm to about 200 µm and is preferably ≥ 45 µm. Undisturbed sapphire and undisturbed titanium are spaced outwardly from the greater than 1 µm diffusion bond, and preferably the ≥ 4 µm diffusion bond.

In still another embodiment, a method for forming a kinetically limited micro-scale diffusion bond having a thickness greater than about 1 µm, and preferably ≥ 4 µm is provided. The method includes positioning a first surface to be bonded of sapphire as an optically transparent ceramic against a second surface to be bonded of titanium, or titanium alloy as an opaque conductive metal. The optically transparent sapphire has properties that allow a Gaussian-Bessel electromagnetic beam of a select wavelength and pulse duration to pass through it without more than minimal energy absorption and the opaque titanium has properties that significantly absorb energy from the electromagnetic beam. Pressure on the sapphire and titanium stack may be applied so that a first surface of the sapphire is in direct intimate contact with a second surface of the titanium. A Gaussian-Bessel electromagnetic beam may then selectively passed through the optically transparent sapphire and directed to the second surface of the opaque conductive titanium. The Gaussian-Bessel electromagnetic beam creates a kinetically limited micro-scale diffusion bond having a thickness that is greater than about 1 µm, and that is preferably ≥ 4 µm between the optically transparent ceramic and the opaque conductive metal.

An exemplary application for the present micro-scale diffusion bond comprises sapphire as a window micro-scale diffusion bonded to a titanium flange in an optical sensor. Optical sensors are useful for detecting the oxygen concentration in the blood of a human or animal and for continuously monitoring the glucose level in a patient, among other applications.

One embodiment of an optical sensor according to the present invention comprises an open-ended container having a housing sidewall extending to a perimeter edge, and a lid secured to the perimeter edge of the housing sidewall to form a device housing defining an enclosed space. The housing sidewall is comprised of titanium and has at least one housing opening communicating with the enclosed space. A titanium flange has opposed flange device and body fluid side surfaces surrounding a flange opening with at least the flange device side surface being planar. A sapphire window has opposed planar window body fluid and device side surfaces. Then, a micro-scale diffusion bond having a thickness that is greater than 1 µm connects the sapphire window body fluid side surface to the flange device side surface aligned with the flange opening, and a weld connects the flange body fluid side surface to an inner surface of the housing sidewall aligned with the at least one housing opening so that the sapphire window diffusion bonded to the titanium flange in turn connected to the housing sidewall hermetically closes the at least one housing opening. The micro-scale diffusion bond connecting the body fluid side surface of the sapphire window to the flange device side surface may be characterized as having been formed using a Gaussian-Bessel laser.

This embodiment of an optical sensor also includes a printed circuit board (PCB) assembly housed inside the enclosed space of the device housing. The PCB assembly comprises a printed circuit board supporting at least an optical sensor that is aligned with the sapphire window and the housing opening, and an electrical energy power source that is configured to power the PCB assembly including the optical sensor.

Importantly, the micro-scale diffusion bond may have a diffusion bond thickness that is ≥ 4 µm as measured along an x-axis aligned perpendicular to a planar contact interface between the sapphire window and the titanium flange with the diffusion bond thickness including the combined depth of the diffusion bond extending from the planar contact interface along the x-axis into the sapphire window and the titanium washer, but not including undisturbed portions of the sapphire window and the titanium washer.

In addition to the micro-scale diffusion bond having the diffusion bond thickness that is ≥ 4 µm as measured along the x-axis aligned perpendicular to the planar contact interface between the sapphire window and the titanium flange, the micro-scale diffusion bond may have a diffusion bond width ranging from about 1 µm to about 200 µm, preferably ≥ 45 µm, extending along a y-axis into the sapphire window and the titanium washer, but not including undisturbed portions of the sapphire window and the titanium flange with the y-axis being aligned along the planar contact interface and intersecting the x-axis at a right angle.

The sapphire window may be optically transparent to electromagnetic (EM) radiation over a range of about 150 nm to about 6,000 nm. The sapphire window may have a thickness extending to the window body fluid and device side surfaces that ranges from 100 µm to 4 mm. The sapphire window may have a material hardness of at least 9 on the Mohs hardness scale.

The micro-scale diffusion bond connecting the sapphire window body fluid side surface to the flange device side surface may be at least one of corrosion-resistant, crack free, uniform, and bio-stable.

Another embodiment of an optical sensor according to the present invention comprises an open-ended container having a housing sidewall extending to an open perimeter such as an annular rim, and a lid secured to the open perimeter to form a housing defining an enclosed space. The housing sidewall is comprised of titanium and has at least one housing opening communicating with the enclosed space. A sapphire window has opposed planar window body fluid and device side surfaces, and a micro-scale diffusion bond having a thickness that is greater than 1 µm connects the sapphire window body fluid side surface to a planar inner surface of the housing sidewall aligned with the at least one housing opening so that the sapphire window diffusion bonded to the housing sidewall hermetically closes the at least one housing opening. There is also a printed circuit board (PCB) assembly housed inside the enclosed space of the device housing. The PCB assembly comprises a printed circuit board supporting at least an optical sensor that is aligned with the sapphire window and the housing opening. An electrical energy power source is configured to power the PCB assembly is housed inside the device housing of the optical sensor.

These and other aspects of the present invention will become increasingly more apparent to those skilled in the art by reference to the following detailed description and to the appended drawings.

### BRIEF DESCRIPTION OF THE DRAWINGS

FIG. 1 is a perspective view of an exemplary optical sensor 10 comprising an open-ended container formed from mating clamshells 14 and 16 closed by a lid 18 according to the present invention.
FIG. 1A is a partial cross-sectional view of another embodiment of a device housing 12A according to the present invention.
FIG. 2 is a partly exploded, perspective view of an optically transparent sapphire window 34 after it has been diffusion bonded to a titanium flange 62 but prior to the assembly being connected on the inner surface 28 of a titanium clamshell 14 portion of the optical sensor 10 shown in FIG. 1.
FIG. 2A is a partly exploded, perspective view of an optically transparent sapphire window 34 prior to it being diffusion bonded directly to the inner surface 28 of a titanium clamshell 14 portion of the optical sensor 10 shown in FIG. 1.
FIG. 3 is a perspective view of the lid 18 for the optical sensor shown 10 in FIG. 1.
FIG. 4 is a schematic illustration of a system 100 for micro-scale diffusion bonding of a ceramic material (sapphire) 34 to titanium 62 using a Gaussian-Bessel laser beam 102 according to the present invention.
FIG. 5 is an exemplary preparation flow diagram 200 for the sapphire window 34 according to the present invention.
FIG. 6 is an exemplary preparation flow diagram 300 for the titanium flange 62 according to the present invention.
FIG. 7 is a flow diagram 400 illustrating the steps for micro-scale diffusion bonding a sapphire window 34 to a titanium flange 62 according to the present invention.
FIG. 8 illustrates the assembly of a stack comprising a sapphire window 34 and a titanium flange 62 between a holding fixture 500 and a sealing cover 504 prior to diffusion bonding according to the present invention.
FIG. 9 is a cross-sectional view along line 9-9 of FIG. 1 illustrating a glass-to-metal seal (GTMS) 54 for the optical sensor 10 with the sealing glass 58 directly hermetically contacting the lid 18 of the device housing 12.
FIG. 9A is a cross-sectional view of an alternate embodiment of a GTMS 54A that is similar to the GTMS 54 shown in FIG. 9 except that the glass 58 seals between a ferule 59 and the terminal pin.
FIG. 10 is a plan view of three circular diffusion bond paths 602, 604 and 606 forming a micro-scale diffusion bonded hermetic seal between a sapphire window 34 and a titanium flange 62 according to the present invention.
FIG. 11 is an optical microscope image of a bonded titanium-sapphire assembly where the beam of EM radiation has formed three separate diffusion bond paths, each having a diffusion bond width of about 45 µm.
FIG. 12 is a cross-sectional scanning electron microscope (SEM) image of a micro-scale diffusion bond that is ≥ 4 µm thick of a titanium-sapphire assembly according to the present invention.
FIG. 13 is a cross-sectional scanning electron microscope (SEM) image of a micro-scale diffusion bond that is greater than 1 µm thick of a titanium-sapphire assembly with sapphire and titanium immediately surrounding the diffusion bond remaining undisturbed.

### DETAILED DESCRIPTION OF THE PREFERRED EMBODIMENTS

In this specification, the term "micro-scale" refers to micro or a micron, which means one millionth (10⁻⁶) of a meter.

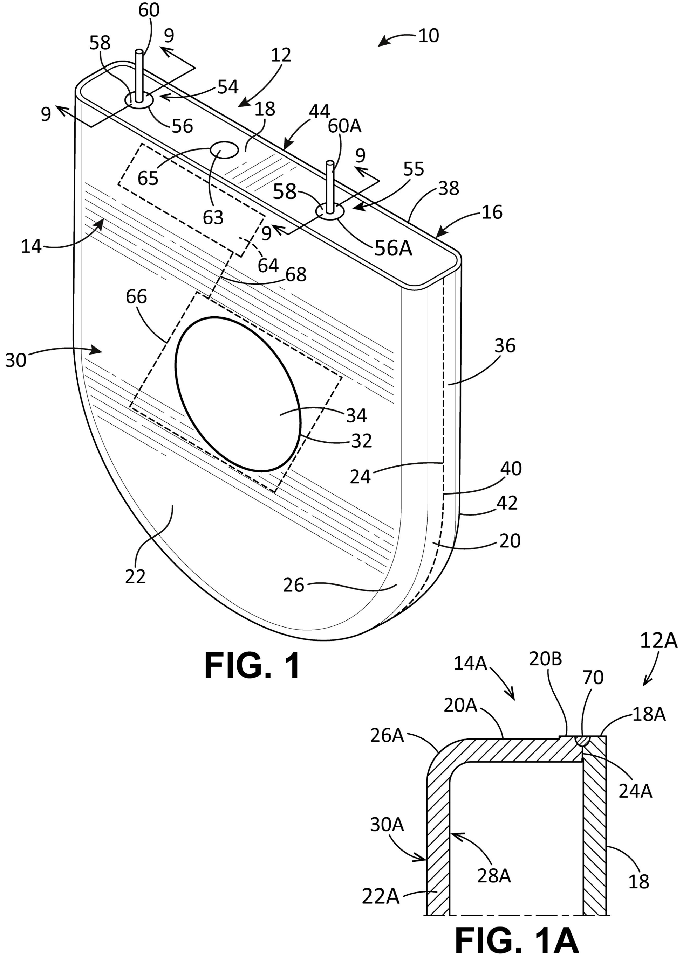

The term "diffusion bond thickness" is measured along an x-axis (see FIG. 1) aligned perpendicular to a planar contact interface between the sapphire window and the titanium flange. The diffusion bond thickness includes the combined depth of the diffusion bond extending from the planar contact interface along the x-axis into the sapphire window and the titanium washer, but not including undisturbed portions of the sapphire window and the titanium washer. In that respect, the diffusion bond thickness is bordered by opposed undisturbed portions of the sapphire window and the titanium workpiece. According to the present invention, the diffusion bond thickness between a sapphire window and a titanium flange is > 1 µm, and preferably ≥ 4 µm.

The term "diffusion bond width" is defined as the micro-scale diffusion bond extending along a y-axis into the sapphire window and the titanium washer aligned along the planar contact interface to intersect the x-axis at a right angle, but not including undisturbed portions of the sapphire window and the titanium flange. In that respect, the diffusion bond width is bordered by opposed undisturbed portions of the sapphire window and the titanium workpiece. According to the present invention, the diffusion bond width between the sapphire window and the titanium flange ranges from about 1 µm to about 200 µm and is preferably ≥ 45 µm.

A Bessel beam is a wave whose amplitude is described by a Bessel function of the first kind. Electromagnetic, acoustic, and matter waves can all be in the form of a Bessel beam. A Bessel beam is non-diffractive within a specific range, known as "depth of field" or "Bessel zone" in the literature. In the specific range, as it propagates, the beam does not diffract and spread out. This contrasts with the usual behavior of light (or sound), which spreads out after being focused down to a small spot. As with a plane wave, a true Bessel beam cannot be created, as it is unbounded and would require an infinite amount of energy. Reasonably good approximations can be made, however, and these are important in many optical applications because they exhibit little or no diffraction over a limited distance. Approximations to Bessel beams are made in practice either by focusing a Gaussian beam with an axicon lens to generate a Gaussian-Bessel beam, by using axisymmetric diffraction gratings, or by placing a narrow annular aperture in the far field.

The term "undisturbed portion" is defined as a portion of the thickness of either the sapphire window or the titanium flange that after creation of the micro-scale diffusion bond remains unaffected by the Gaussian-Bessel beam.

Turning now to the drawings, FIG. 1 illustrates an optical sensor 10 according to the present invention. The optical sensor 10 includes a hermetically sealed device housing 12 comprising an open-ended container of an electrically conductive material, such as titanium, titanium alloys or stainless steel. The open-ended container is formed from mating first and second clamshells 14 and 16 that are seam-welded to each other. The open-ended container is closed by a lid 18 to form the device housing 12 defining an enclosure space for the optical sensor 10.

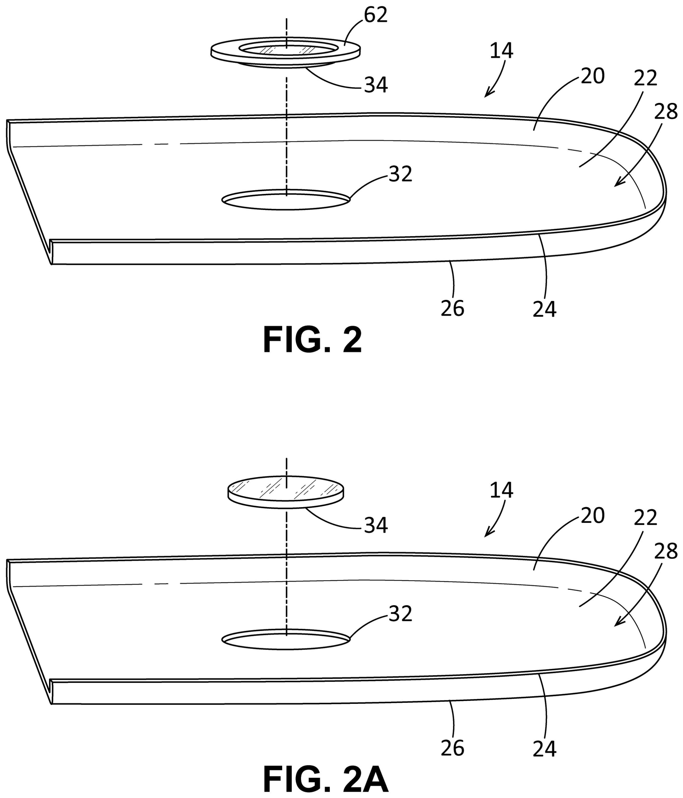

As further shown in FIGS. 1 and 2, the first clamshell 14 has a first U-shaped sidewall 20 that extends upwardly from a front or first major face wall 22 to a first rim 24 of the sidewall. A portion of the first major face wall 22 that is not connected to the first U-shaped sidewall 20 resides between opposed terminal ends of the sidewall 20. A first rounded or curved edge 26 connects the first sidewall 20 to the first major face wall 22. The first rim 24 is spaced from a planar inner surface 28 of the first major face wall 22 (FIG. 2). The first major face wall 22 also has a planar outer surface 30 that is opposed to the inner surface 28. The distance between the planar inner and outer surfaces 28, 30 defines the thickness of the first clamshell 14. The first U-shaped sidewall 20 connected to the first major face wall 22 by the intermediate first rounded or curved edge 26 collectively comprise the sidewall forming the first clamshell 14.

The first major face wall 22 has an opening 32 through its thickness. According to the present invention, a sapphire window 34 that is optically transparent to electromagnetic (EM) radiation over a range of about 150 nm to about 6,000 nm is supported on the inner surface 28 of the first major face wall 22 in alignment with the opening 32. The sapphire window 34 has a thickness extending to its body fluid and device side surfaces that ranges from 100 µm to 4 mm, and a material hardness of about 9 on the Mohs hardness scale.

FIG. 1 also illustrates the second clamshell 16 of the device housing 12 for the optical sensor 10. In a similar manner as the first clamshell 14, the second clamshell 16 has a second U-shaped sidewall 36 that extends upwardly from a back or second major face wall 38 to a second rim 40 of the sidewall. A portion of the second major face wall 38 that is not connected to the second U-shaped sidewall 36 resides between opposed terminal ends of the sidewall 36. A second rounded or curved edge 42 connects the second sidewall 36 to the second major face wall 38. The second rim 40 is spaced from a planar inner surface (not shown) of the second major face wall 38. The second major face wall 38 has a planar outer surface 44 that is opposed to the inner surface. The distance between the planar inner and outer surfaces defines the thickness of the second clamshell 14. The second U-shaped sidewall 36 connected to the second major face wall 38 by the intermediate second rounded or curved edge 42 collectively comprise the sidewall forming the second clamshell 16.

**In** one embodiment, the mating clamshells 14 and 16 are stamped or otherwise formed from sheet metal, for example, titanium, a titanium alloy, or stainless-steel sheet stock. This construction technique readily allows for the provision of the opening 32 in the first major face wall 22 of the first clamshell 14. However, it should be readily apparent to those skilled in the art that the shape of the first and second clamshells 14, 16, and the shape, diameter, and exact positioning of the opening 32 in the first clamshell 14 is by way of example only. **In** a broader sense, either one or both of the first and second clamshells 14 and 16 can have different complementary shapes than what is shown and the first and second clamshells 14 and 16 can have a single opening or a plurality of openings formed into their respective major face walls 22 and 38 anywhere within their planar surface areas.

To form the open-ended container comprising the device housing 12 for the optical sensor 10, the first rim 24 of the first clamshell 12 is aligned with and butted against the second rim 40 of the second clamshell 16. The thusly mated first and second clamshells 14 and 16 are then seam-welded together at the butted rims 24 and 40. A laser beam (not shown) emitted from a laser source is a preferred welding technique. Welding two butted workpieces together using a laser beam is well known by those skilled in the art.

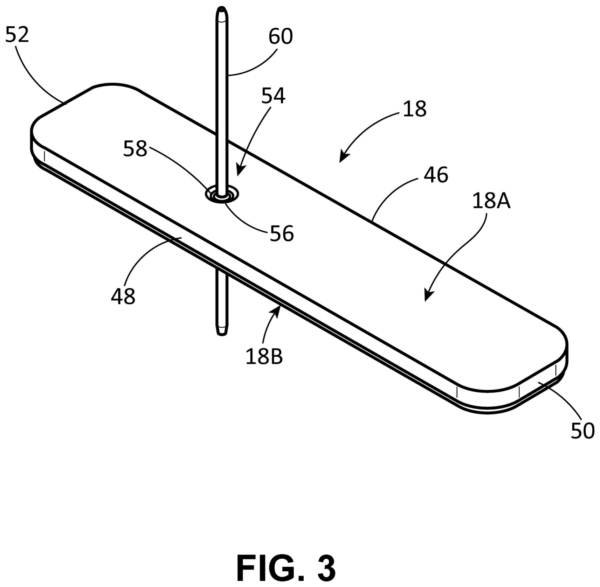

The lid 18 is shown in greater detail in FIG. 3 having a peripheral edge comprising spaced apart upper and lower side edge portions 46 and 48 extending to opposed right and left end edge portions 50 and 52. There are curved corners where the side edge portions 46, 48 meet the end edge portions 50, 52. The side edge portions 46, 48 and the end edge portions 50, 52 and the included curved corners extend to and meet with an outer lid surface 18A spaced from an inner lid surface 18B. Preferably the outer and inner lid surfaces 18A and 18B are planar and aligned parallel to each other.

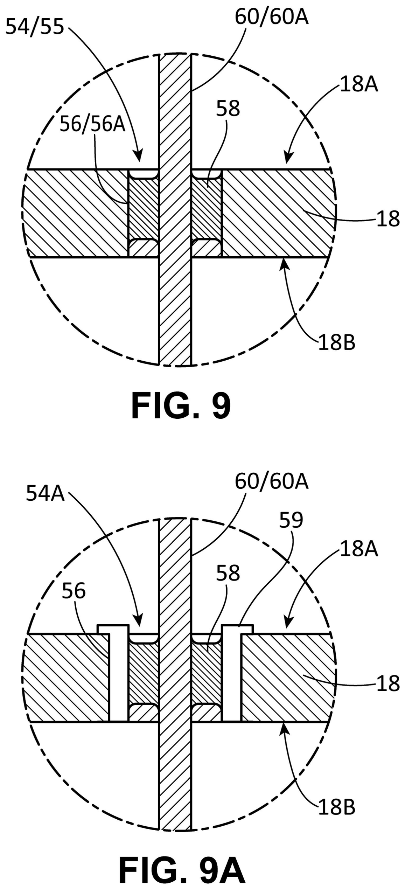

FIGS. 3 and 9 further illustrate that the lid 18 supports two glass-to-metal seals (GTMS) 54 and 55. The GTMSs 54 and 55 comprise respective openings 56, 56A that extend through the thickness of the lid 18 from its outer to inner surfaces 18A, 18B. In this embodiment, the GTMSs 54 and 55 do not have a ferrule. Instead, a sealing glass 58 directly hermetically contacts the lid 18 at the perimeter of the opening 56, 56A and directly hermetically contacts a terminal pin 60, 60A to electrically isolate the terminal pin from the lid 18 closing the open-ended container forming the device housing 12. In that manner, the terminal pins 60, 60A serve as hermetically sealed electrically conductive pathways extending from a device side of the pins residing inside the device housing 12 to a body fluid side of the terminal pins 60, 60A extending outwardly from the device housing. Moreover, while two terminal pins 60, 60A are shown in the drawings, it is within the scope of the present invention that there can be many more hermetically sealed terminal pins extending through the lid 18. The number of terminal pins is a function of the specific application in which the optical sensor 10 is designed to operate. Further, the terminal pins 60, 60A can be active or ground terminal pins.

FIG. 9A illustrates an alternate embodiment of a GTMS 54A comprising a ferrule 59 that is welded into the lid opening 56. The ferrule 59 provides an opening and the sealing glass 58 directly hermetically contacts the inner surface of the ferrule in the opening and directly hermetically contacts the terminal pin 60, 60A to electrically isolate the terminal pin from the ferrule connected to the lid 18 closing the open-ended container comprising the device housing 12.

A suitable sealing glass 58 is selected from Ferro IP510, Corning 1890, Schott 8422, Schott 8629, TA-23, which is an alkaline earth aluminosilicate type glass developed by Sandia National Labs with a melting temperature of about 775° C., and Cabal-12, which is a calcium-boro-aluminate type glass that was also developed by Sandia National Labs with a melting temperature of about 925° C.

In lieu of or in addition to the GTMSs 54 and 55 supporting the respective terminal pins 60, 60A, FIG. 1 further illustrates that the lid 18 can support an electrically conductive pathway comprising a platinum-containing material 63 filled into a via hole 65 extending through the thickness of the lid. For example, the platinum-containing material 63 can be a substantially closed pore, fritless and substantially pure platinum material that fills the via hole 65. The conductive pathway 63 can be an active or ground conductive pathway, and in a similar manner as the previously described terminal pins 60, 60A, the lid 18 can support two or many more conductive pathways.

In lieu of the platinum-containing material 63 being a substantially pure platinum material, the via hole 65 is filled with a composite reinforced metal ceramic (CRMC) material. The CRMC material is not a substantially pure platinum material, but comprises, by weight %, from about 10:90 ceramic: platinum to about 90:10 ceramic: platinum or, from about 70:30 ceramic: platinum to about 30:70 ceramic: platinum. Examples of suitable ceramic materials for the CRMC include, but are not limited to, alumina (Al₂O₃) or zirconia (ZrO₂) including various stabilized or partially stabilized zirconia, for example, zirconia toughened alumina (ZTA) and alumina toughened zirconia (ATZ) with platinum (Pt) or palladium (Pd).

Preferably, the platinum-containing material 63, whether it is a substantially pure platinum material or the CRMC material, is in the form of a paste having a platinum particle powder or platinum/ceramic particle powder loading ranging from about 20 volume % to about 90 volume % and a viscosity ranging from about 1 x 10⁵ cP to about 1 x 10¹⁰ cP. The platinum-containing material 63 as a paste is a mixture of a substantially pure platinum powder or a platinum/ceramic particle powder, an inactive organic binder, and possibly a solvent and/or plasticizer. Suitable binders are selected from the group consisting of ethyl cellulose, acrylic resin, polyvinyl alcohol, polyvinyl butyral, and a poly(alkylene carbonate) having the general formula R-O-C(=O)-O with R=C₁ to C₅. Poly(ethylene carbonate) or poly(propylene carbonate) are preferred poly(alkylene carbonates). Suitable solvents are selected from the group consisting of terpineol, butyl carbitol, cyclohexanone, n-octyl alcohol, ethylene glycol, glycerol, water, and mixtures thereof.

Suitable methods for filling the active or ground via hole 65 with a paste of platinum-containing material 63 include a vacuum pull, a pressure push, a squeegee fill, among other techniques. In addition, the active or ground via hole 63 must be packed so that the platinum-containing paste occupies at least about 90% of its available space. In a preferred embodiment, the platinum-containing paste occupies about 95% of the available space in the active or ground via hole 65. In a more preferred embodiment, the platinum-containing paste occupies about 99% of the available space in the active/ground via hole 65.

For additional information regarding via holes filled with electrically conductive materials, reference is made to U.S. Patent Nos. 8,653,384 to Tang et al., 9,492,659 to Tang et al., 10,249,415 to Seitz et al. and RE47,624 to Tang et al. (which is a re-issue of the '384 patent). These patents are assigned to the assignee of the present invention and incorporated herein by reference. For additional information regarding via holes filled with a CRMC material, reference is made to U.S. Patent Nos. 10,350,421 to Seitz et al. and 10,272,252 to Seitz et al. These patents are also assigned to the assignee of the present invention and incorporated herein by reference.

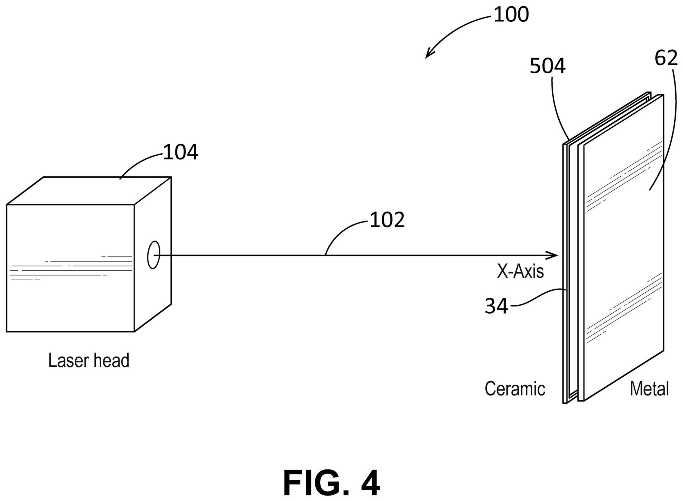

Referring now to FIG. 4, a system 100 for micro-scale diffusion bonding dissimilar bulk materials using perpendicularly incident electromagnetic (EM) radiation, for example, a Gaussian-Bessel laser beam, at room temperature, according to the present invention is illustrated. In general, the materials being micro-scale diffusion bonded include the optically transparent sapphire window 34 (ceramic wafer) and an electrically conductive and opaque metallic metal, for example, a titanium or titanium alloy flange 62 (FIG. 2) in the shape of a washer. More particularly, sapphire as a single crystalline Al₂O₃ is a preferred material for the window 34 because it is optically transparent to the wavelength of a laser beam 102, preferably a Gaussian-Bessel beam, emitted from the laser head 104. The optically transparent sapphire window 34 has properties that allow the laser beam 102 to pass through it with nothing more than minimal light reflection at its front surface.

Further, the sapphire window 34 has opposed window body fluid and device side surfaces which are interchangeable but are used as nomenclature to indicate that when the window is connected to the titanium flange 62, the window device side surface 34A will face the enclosed space inside the device housing 12 and the opposed window body fluid side surface will face outside the device housing.

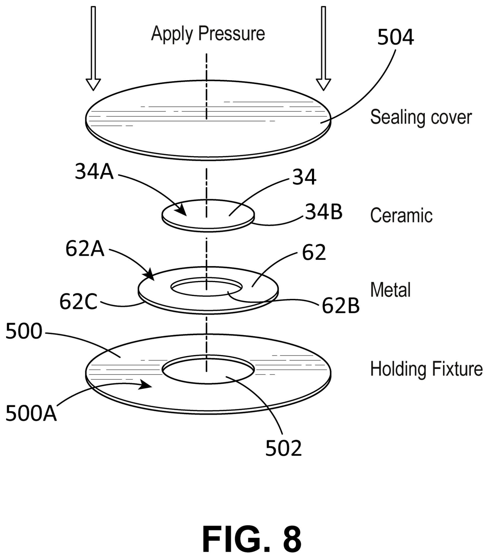

Titanium is a preferred electrically conductive and opaque metal for the flange 62. The flange 62 has opposed flange device and body fluid side surfaces. The flange device and body fluid side surfaces are interchangeable but are used as nomenclature to indicate that when the flange is connected to the planar inner surface 28 of the first clamshell 14, the flange device side surface will face the enclosed space inside the device housing 12 and the opposed flange body fluid side surface 62A will face outside the device housing. The flange 62 has an opening 62B (FIG. 8).

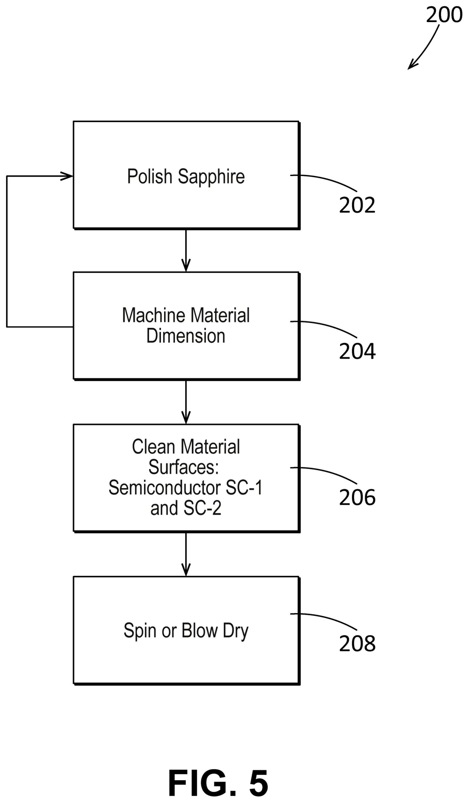

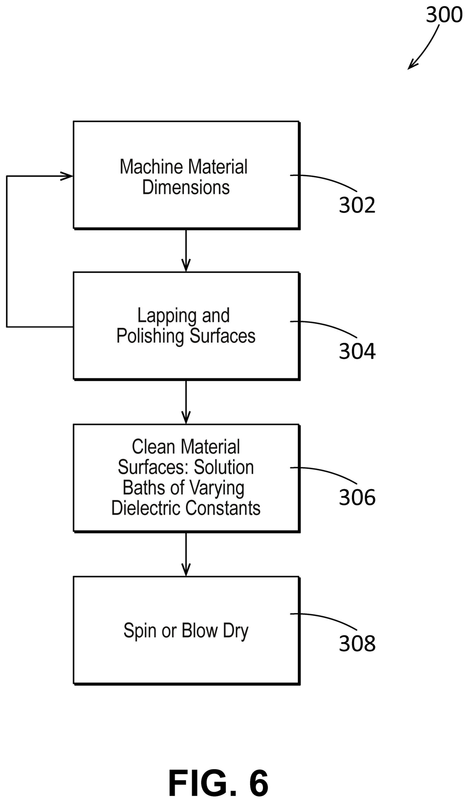

Prior to micro-scale diffusion bonding the sapphire and titanium materials 34, 62 using the Gaussian-Bessel laser beam 104 in the system 100 depicted in FIG. 4, the bulk materials are processed to prepare them to be bonded together. The steps for preparation of the sapphire-titanium materials 34, 62 are depicted in the sapphire preparation flow diagram 200 shown in FIG. 5 and the titanium preparation flow diagram 300 shown in FIG. 6.

Regarding sapphire, in step 202 of the flow diagram 200 shown in FIG. 5, the sapphire window 34 is first polished. In step 204, the polished sapphire window 34 is then diced to size, as required. If desired, the sapphire window 34 is sent back to step 202 for further polishing before again moving to step 204 for machining to a desired dimension.

As depicted in step 206 of the flow diagram shown in FIG. 5, once the desired material dimensions and surface quality are obtained, the sapphire window 34 is cleaned in a series of solution baths of increasing dielectric constant. One such embodiment subjects the sapphire window 34 to baths of hexanes, acetone, ethanol, and then deionized water. In other embodiments, the sapphire window 34 is subjected to standard semiconductor cleaning processes. One such embodiment subjects the sapphire window 34 to chemical baths referred to in the industry as Standard Clean 1 (SC-1) and Standard Clean 2 (SC-2) before rinsing with deionized water. As depicted in step 208, after the sapphire window 34 is cleaned, it is spun or blown dry. Importantly, wiping the prepared sapphire window 34 is avoided.

Regarding the titanium preparation flow diagram 300 shown in FIG. 6, the flange 62 having the shape of the washer shown in FIG. 2 of a suitable grade of titanium is first selected. In step 302, the titanium flange 62 is machined or stamped to form planar and parallel inner and outer or device side and body fluid side surfaces. One of the planar surfaces will be micro-scale diffusion bonded to the sapphire window 34 and the other provides a relatively flat surface upon which pressure can be applied. In an alternate embodiment, only the surface of the titanium flange 62 that is intended to be diffusion bonded to the sapphire window 34 is machined planar. A conforming fixture will provide a relatively uniform surface to which pressure to the second side of the flange 62 can be applied.

To improve surface quality, in step 304, a lapping and polishing process is applied to at least the planar surface of the titanium flange 62 that is intended to be bonded to the sapphire window 34. Lapping includes the use of a combination of Blanchard grinding and a 12 µm diameter aluminum oxide (Al₂O₃) slurry (alumina slurry). In an alternate embodiment to improve surface quality, the bonding surface of the titanium flange 62 is polished in a two-step process. The first polishing step uses a mixture of a 1.5 µm diamond slurry. The second and final polishing step uses a 0.5 µm diamond slurry.

Regardless the lapping and polishing process used, flatness or planarity in the sub-micron range is important to ensure intimate contact of the sapphire and titanium surfaces to be bonded when they are mated. A smooth, scratch free surface with a roughness (Ra) of less than 60 nm is desirable to ensure intimate contact at the sapphire-titanium interface during micro-scale diffusion bonding. However, a hermetic micro-scale diffusion bond can be achieved with a roughness up to 200 nm or greater.

As depicted in step 306 of the flow diagram shown in FIG. 6, once the desired material dimensions and surface quality are obtained, the titanium flange 62 is cleaned. In one embodiment, the titanium is washed with soapy water and then rinsed with deionized water. In other embodiments, the titanium flange 62 is subjected to a series of solution baths of increasing dielectric constant. One such embodiment subjects the titanium flange 62 to baths of hexanes, acetone, ethanol, and then deionized water. As depicted in step 308, after the titanium flange 62 is cleaned, it is spun or blown dry. Importantly, wiping the prepared titanium flange 62 is avoided.

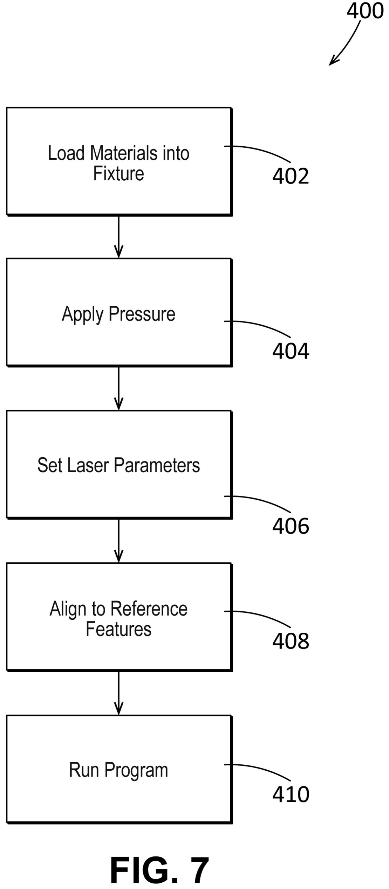

As shown in the flow diagram 400 in FIG. 7, after the sapphire window 34 and the titanium flange 62 have been prepared for micro-scale diffusion bonding, they are loaded into a holding fixture in step 402. FIG. 8 illustrates the holding fixture as a plate-shaped member 500 having a central recess 502. The central recess 502 is sized and shaped to receive the prepared titanium flange 62. Preferably, the recess 502 is only slightly larger in diameter than the perimeter of the titanium flange 62 so that the flange 62 is prevented from moving along an x-axis or a y-axis with respect to the fixture 500. With the titanium flange 62 received in the fixture recess 502, an upper surface 500A of the fixture 500 is spaced a very short distance below a device side surface 62A of the titanium flange 62. The sapphire window 34 is then moved into a stacked position contacting the device side surface 62A of the titanium flange 62. An optically transparent sealing cover 504 is then moved into direct contact with a device side surface 34A of the sapphire window 34. The sapphire window also has an opposed body fluid side surface.

As depicted in step 404 of the flow diagram shown in FIG. 7, once the sapphire window 34 and the titanium flange 62 are properly positioned in the holding fixture 500 with the desired bonding surfaces in direct contact with each other and with the optically transparent sealing cover 504 directly contacting the sapphire window 34, an external pressure is applied to the stack. This external pressure increases the intimacy and uniformity of contact between the sapphire and titanium surfaces. The magnitude of pressure needed to form a micro-scale diffusion bond is at least partially dependent on the chemical composition of the materials to be bonded, the size and shape of the materials to be bonded, and the surface quality of the materials to be bonded. Once the sapphire window 34 and the titanium flange 62 have been properly stacked in the holding fixture 500 and external pressure has been applied to the stack, the laser parameters are set. This is depicted in step 406 of the flow diagram shown in FIG. 7.

As illustrated in FIG. 4, a Gaussian-Bessel beam 102 of electromagnetic (EM) radiation emanating from a laser head 104 is directed through the optically transparent sealing cover 504 (FIG. 8) and the sapphire window 34 to the interface between the sapphire and titanium materials, preferably at room temperature. In one embodiment according to the present invention, the laser bonding parameters for a Gaussian-Bessel beam 102 to diffusion bond the sapphire and titanium materials together include a bonding pressure ranging from about 5 psi to about 30 psi, a pulse beam wavelength of about 1,030 nm in the near infrared range, a pulse energy of about 16 µJ, a pulse duration (width) of about 400 femtoseconds (fs), a pulse frequency of about 400kHz, a laser beam spot size of about 2 µm with the speed of laser movement ranging from about 5 mm/sec. to about 600 mm/sec. However, it is within the scope of the present invention that selecting a specific unique value for one parameter will result in a specific unique value for another parameter. Selection of a different set of specific unique values may form a resulting micro-scale diffusion bond that is statistically identical in observable properties. Accordingly, the invention is not limited to specific parameters.

Once the operating laser parameters are set in step 406 of the flow diagram shown in FIG. 7, the Gaussian-Bessel laser is aligned to reference features in step 408. In one embodiment, the reference features refer only to an edge of the stacked sapphire and titanium materials. In another embodiment, the reference features refer to a geometric shape inscribed on the sapphire and titanium bonding surfaces. As depicted in step 410 of the flow diagram shown in FIG. 7, a program is then run by a controller connected to the laser head 104 (FIG. 4) to activate the Gaussian-Bessel beam 102 of EM radiation and enable the motorized stage to either move the holding fixture 500 or the laser head 104 along the desired bonding path in the x, y, and z directions.

The energy passing through the optically transparent sapphire 34 outside the Bessel zone to subsequently interact with the optically absorbent titanium 62 to form the desired micro-scale diffusion bond generally needs to meet the following requirements: 1) the energy transmitted through the optically transparent sapphire 34 must be sufficient to activate the bonding process at the sapphire/titanium interface via absorption by the opaque titanium 62 within the Bessel zone (the laser energy will not be absorbed and interact with the sapphire and the titanium until it gets into the Bessel zone because the laser intensity is only strong enough within this zone to generate a sufficiently strong absorption), and simultaneously 2) any energy absorbed by the optically transparent sapphire 34 must not be sufficient to melt, distort, or otherwise affect the bulk of the sapphire 34 spaced outwardly from the bond interface.

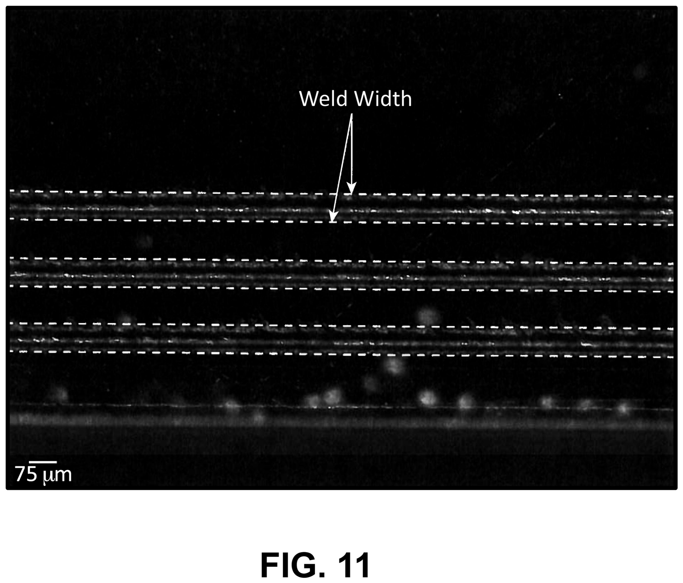

Thus, the optically transparent sapphire 34 and the optically absorbent titanium 62 absorb and interact with the energy of the Gaussian-Bessel laser beam 102 within the Bessel zone so that an interfacial bond (FIGS. 11 to 13) is created between them by diffusion of the optically transparent sapphire 34 into the opaque titanium 62, by diffusion of the opaque titanium 62 into the optically transparent sapphire 34, and by diffusion of both materials into each other. The resulting micro-scale diffusion bond has a thickness that is > 1 µm, and preferably ≥ 4 µm, as measured along an x-axis aligned perpendicular to a planar contact interface between the sapphire window and the titanium flange. The diffusion bond thickness includes the combined depth of the diffusion bond extending from the planar contact interface along the x-axis into the sapphire window and the titanium washer, but does not include undisturbed portions of the sapphire window and the titanium washer. A diffusion bond that is at least 4 µm thick is desired to bond the dissimilar sapphire and titanium bulk materials 34, 62 together in a hermetic connection that is suitable for long-term implantation into a human or animal body.

**In** that respect, the micro-scale diffusion bond has an interfacial toughness (strength) that is similar or equivalent to the strength of at least one of the bulk materials 34 and 62. Moreover, the micro-scale diffusion bond does not contain cracks or imperfections that are large enough to reduce the measured fracture toughness of the diffusion bond to a degree below that of the bulk fracture toughness of the optically transparent sapphire 34. Desirably, the micro-scale diffusion bond is generally continuous, uniform, and crack free. Importantly, the micro-scale diffusion bond provides a hermetic seal between the bulk materials 34, 62 that is corrosion resistant and bio-stable. Further, the diffusion bond is a relatively thin interface in the micro-scale range due to a short heating time. Due to the relatively short local bonding time and the absence of bulk heating, the formation of undesirable compounds at or near the sapphire/titanium interface that could weaken the micro-scale diffusion bond is minimized or eliminated.

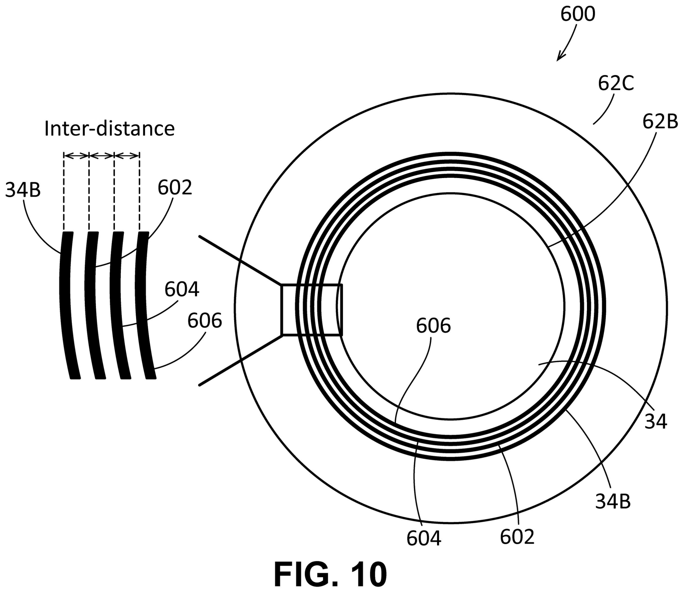

FIG. 10 illustrates one exemplary diffusion bond pattern according to the present invention. In this drawing, the stacked assembly 600 comprises the sapphire window 34 pressed against the device side surface 62A of the titanium flange 62. This drawing shows the outer edge 34B of the sapphire window 34 contacting the device side surface 62A of the titanium flange 62 with the window 34 being substantially centered in alignment with the flange opening 62B. The outer edge 62C of the flange 62 is also illustrated.

There are three concentric circularly-shaped weld paths forming diffusion bonds 602, 604 and 606 of increasingly lesser diameters forming a diffusion bond pattern hermetically sealing the sapphire window 34 to the titanium flange 62. The resulting diffusion bonds 602, 604 and 606 each have a diffusion bond width that ranges from about 45 µm to as large as 200 µm with a diffusion bond penetration or diffusion bond thickness that is greater than about 1 µm to about 10 µm. The diffusion bonds do not contain cracks or imperfections large enough to observe with an optical microscope. The distance between the outer edge 34B of the sapphire window 34 and the outer-most diffusion bond path 602 ranges from about 100 µm to about 1,000 µm, and the distance between the respective diffusion bonds 602, 604 and 606 ranges from about 100 µm to about 500 µm.

FIG. 11 depicts an optical microscope image of a bonded sapphire-titanium assembly wherein the beam of EM radiation 102 from the Gaussian-Bessel laser 104 (FIG. 4) formed three separate diffusion bond paths. The image in FIG. 11 is collected at 90° incidence to the sapphire-titanium interface, observing the bonding paths through the sapphire component of the bonded assembly. As illustrated, each diffusion bond formed because of EM radiation from the Gaussian-Bessel laser 104 comprises three linear features: two semi-straight lines which blur or fuzz at intervals and border a third straight line. As illustrated in the sapphire-titanium example, the diffusion bond width across a single path is uniformly greater than 45 µm.

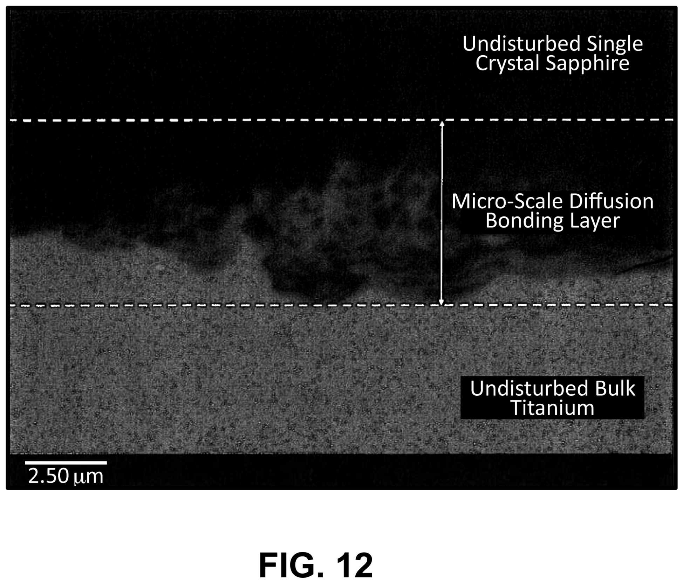

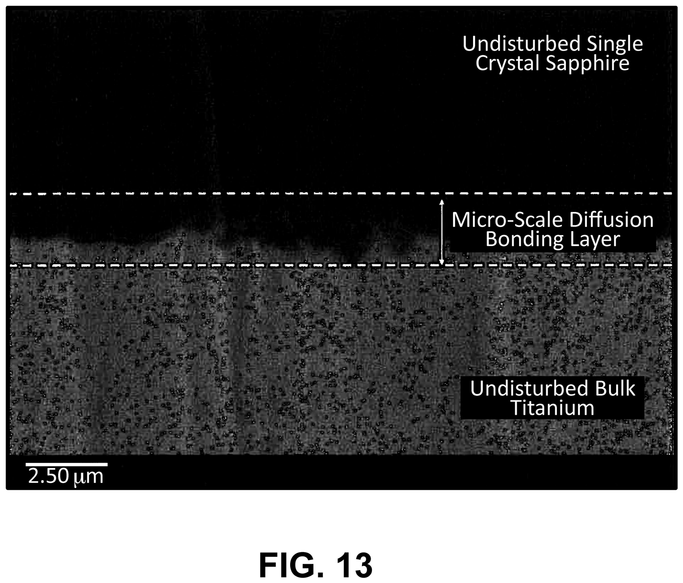

FIGS. 12 and 13 depict a cross-sectional scanning electron microscope (SEM) image of a diffusion zone in a bonded sapphire-titanium assembly as formed using a Gaussian-Bessel laser 104 (FIG. 4) programmed with the parameters discussed above. As a result of the beam of incident EM radiation 102, titanium has diffused into the sapphire to form a diffusion zone as a plume or cloud-like insertion of titanium into the sapphire material that is greater than 1 µm thick. Particularly, the diffusion zone is about 6 µm thick in the image shown in FIG. 12 and about 2 µm thick in the image shown in FIG. 13. In the areas immediately surrounding the cloud-like diffusion layers, both the sapphire and titanium are undisturbed. That is, the formation of the diffusion bond, as described above, does not change the properties of the sapphire and titanium beyond the localized diffusion zone. Thus, a micro-scale diffusion bond having a thickness that is greater than about 1 µm, and preferably ≥ 4 µm, connecting sapphire as an optically transparent insulating ceramic to titanium as an opaque conductive metal has been described.

Referring back to FIGS. 1 and 2, the sapphire window 34 diffusion bonded to the titanium flange 62 is now ready to be connected to the titanium clamshell 14. FIG. 2 shows the sapphire window 34/titanium flange 62 assembly being moved toward the inner surface 28 of the clamshell 14. Preferably, the outer diameter of the outer edge 34B of the sapphire window 34 is only slightly less than the diameter of the opening 32 in the clamshell 14, and the outer surface of the window 34 is flush with the planar outer surface 30 of the clamshell 14. The titanium flange 62 in then welded to the inner surface 28 of the clamshell 14 at its outer edge 62C. This weld in conjunction with the hermetic diffusion bond of the sapphire window 34 to the titanium flange 62 creates a hermetic connection of the sapphire window 34/titanium flange 62 assembly to the clamshell 14.

FIG. 2A illustrates an alternate embodiment where the optically transparent sapphire window 34 is diffusion bonded directly to the inner surface 28 of the titanium clamshell 14 portion of the optical sensor 10 shown in FIG. 1. In this embodiment, the titanium washer 72 has been eliminated.

FIG. 1 further illustrates in phantom that an electrical energy power source 64 is housed inside the device housing 12. The power source 64 can be a capacitor or a rechargeable battery, for example a hermetically sealed rechargeable Li-ion battery. However, the power source 64 is not limited to any one chemistry or even a rechargeable chemistry and can be of an alkaline cell, a primary lithium cell, a rechargeable lithium-ion cell, a Ni/cadmium cell, a Ni/metal hydride cell, a supercapacitor, a thin film solid-state cell, and the like. Preferably, the power source 64 is a lithium-ion electrochemical cell comprising a carbon-based or Li₄Ti₅O₁₂-based anode and a lithium metal oxide-based cathode, such as of LiCoO₂ or lithium nickel manganese cobalt oxide (LiNiₐMn_{b}Co_{1-a-b}O₂). The power source 64 can also be a solid-state thin film electrochemical cell having a lithium anode, a metal-oxide based cathode and a solid electrolyte, such as an electrolyte of LiPON (LiₓPO_{y}N_{z}).

FIG. 1 further illustrates in phantom that a printed circuit board (PCB) assembly 66 comprising at least one electronic component electrically connected to a printed circuit board is also housed inside the device housing 12. The PCB assembly 66 is connected to the power source 64 by a circuit trace 68. Importantly, the PCB assembly 66 includes an optical sensor component that is configured to detect one or more external optical signals in the form of electromagnetic (EM) radiation over a range of about 150 nm to about 6,000 nm. This EM radiation sensing range coincides with the optical transparency of the sapphire window 34. In that manner, the optical signals pass through the optically transparent sapphire window 34 to impinge on the optical sensor component of the PCB assembly 66 with nothing more than minimal light reflection at the body fluid side surface of the window 34. The electromagnetic radiation incident on the optical sensor component is processed by the PCB assembly 66 to detect the oxygen level in the blood of a human or animal body in which the optical sensor 10 is implanted. The optical sensor 10 is also useful for continuously monitoring the glucose level in a body, among other applications.

The terminal pins 60, 60A, and/or alternately the two or more conductive pathways 63, are connected to the PCB assembly 66. That way, the oxygen level of a patient in which the oxygen sensor 10 of the present invention is implanted can be transmitted out of the sensor 10 for further processing by a computer into a format that is useful to an attending physician.

Alternatively, the terminal pins 60, 60A and/or the conductive pathway 63 are not needed. Instead, the detected optical signals from a human or animal body that pass through the optically transparent sapphire window 34 to impinge on the optical sensor component of the PCB assembly 66 are processed by the PCB assembly 66 and then transmitted as an electromagnetic signal back through the sapphire window 32 for further processing by a computer into a format that is useful to an attending physician.

The PCB assembly 66 also supports an inductive charging antenna (not shown) connected to a charging circuit (not shown). The charging circuit is configured to convert RF or inductive energy signals received by the inductive charging antenna from an external source into a direct current voltage to charge the power source 64 to power the electronic components of the PCB assembly 66. Since the charging antenna will not contact body fluids, it can be made from a less expensive, non-biocompatible material, for example, copper. For a more thorough discussion of a suitable charging antenna, reference is made to U.S. Patent No. 12,434,064 to Barbat. This patent is assigned to the assignee of the present invention and incorporated herein by reference.

Once the power source 64 connected to the PCB assembly 66 are positioned inside the open-ended container, the lid 18 is fitted over the open end thereof. In a preferred embodiment, the perimeter edge of the lid 18 is positioned within the open perimeter of the open-ended container formed by the seam-welded clamshells 14, 16. Alternatively, the lid 18 is positioned with its inner surface 18B contacting an upper edge of the open perimeter. In any event, the lid 18 is preferably welded to the open perimeter of the container, preferably by a laser weld (not shown), to close the container and thereby provide the device housing 12 for the optical sensor 10.

Referring now to FIG. 1A, another embodiment of a device housing 12A that is suitable for practicing the present invention is shown. The device housing 12A comprises a single clamshell 14A having a continuous sidewall 20A that extends upwardly from a major face wall 22A to an annular rim 24A forming an open perimeter of the sidewall. A rounded or curved edge 26A connects the sidewall 20A to the major face wall 22A. The rim 24A is spaced from a planar inner surface 28A of the major face wall 22A. A planar outer surface 30A is opposed to the inner surface 28A, and the distance between the planar inner and outer surfaces 28A, 30A defines the thickness of the clamshell 14A. The U-shaped sidewall 20A connected to the major face wall 22A by the intermediate rounded or curved edge 26A collectively comprise the sidewall forming the clamshell 14A. To close the open perimeter defined by the rim 24A of the clamshell 14A, the lid 18 is rested on the rim 24A so that their respective outer edges 18A and 20B are coplanar. A weld 70 is formed at the interface between the edges to hermetically secure the housing members 14A, 18 together.

In a similar manner as with the device housing 12 shown in FIG. 1, the major face wall 22A of the clamshell 14A is provided with at least one opening (not shown). The previously described titanium flange 62 comprising opposed flange device and body fluid side surfaces surrounding a flange opening with at least the flange device side surface being planar is diffusion bonded to the sapphire window 34. The sapphire window has opposed planar window body fluid and device side surfaces, and the micro-scale diffusion bond connecting the sapphire window body fluid side surface to the flange device side surface aligned with the flange opening has a thickness that is greater than 1 µm. A weld (not shown) then connects the flange body fluid side surface to an inner surface 28A of the major face wall 22A of the clamshell 14A aligned with the at least one housing opening so that the sapphire window diffusion bonded to the titanium flange in turn connected to the clamshell 14A hermetically closes the at least one opening in the clamshell 14A.

In still another alternate embodiment, the body fluid side surface of the sapphire window is micro-scale diffusion bonded to a planar inner surface of the clamshell 14A aligned with the at least one opening in the clamshell. That way, the sapphire window diffusion bonded to the clamshell 14A hermetically closes the at least one clamshell opening. As with the optical sensor 10 shown in FIG. 1, this embodiment of an optical sensor according to the present invention also has a printed circuit board (PCB) assembly comprising a printed circuit board supporting at least an optical sensor. The optical sensor is aligned with the sapphire window and the housing opening. There is also an electrical energy power source that is configured to power the PCB assembly including the optical sensor.

In a further embodiment, the lid 18 is provided with an opening, and the sapphire window/titanium flange diffusion bonded assembly is welded to an inner surface of the lid aligned with the lid opening. Furthermore, the titanium flange is eliminated and the sapphire window is diffusion bonded to the inner surface of the lid 18 aligned with the lid opening.

It is appreciated that various modifications to the inventive concepts described herein may be apparent to those skilled in the art without departing from the spirit and scope of the present invention as defined by the hereinafter appended claims.

## Claims

1. An optical sensor, comprising:
a) an open-ended container comprising a housing sidewall extending to a perimeter edge;
b) a lid secured to the perimeter edge of the housing sidewall to form a device housing defining an enclosed space, wherein the housing sidewall is comprised of titanium and has at least one housing opening communicating with the enclosed space;
c) a titanium flange comprising opposed flange device and body fluid side surfaces surrounding a flange opening, wherein at least the flange device side surface is planar;
d) a sapphire window comprising opposed planar window body fluid and device side surfaces, wherein a micro-scale diffusion bond having a thickness that is greater than 1 µm connects the sapphire window body fluid side surface to the flange device side surface aligned with the flange opening, and wherein a weld connects the flange body fluid side surface to an inner surface of the housing sidewall aligned with the at least one housing opening so that the sapphire window diffusion bonded to the titanium flange in turn connected to the housing sidewall hermetically closes the at least one housing opening;
e) a printed circuit board (PCB) assembly housed inside the enclosed space of the device housing, the PCB assembly comprising a printed circuit board supporting at least an optical sensor, wherein the optical sensor is aligned with the sapphire window and the housing opening; and
f) an electrical energy power source that is configured to power the PCB assembly including the optical sensor.

2. The optical sensor of claim 1, wherein the micro-scale diffusion bond has a diffusion bond thickness that is ≥ 4 µm as measured along an x-axis aligned perpendicular to a planar contact interface between the sapphire window and the titanium flange, and wherein the diffusion bond thickness includes the combined depth of the diffusion bond extending from the planar contact interface along the x-axis into the sapphire window and the titanium washer, but not including undisturbed portions of the sapphire window and the titanium washer,
optionally wherein in addition to the micro-scale diffusion bond having the diffusion bond thickness that is ≥ 4 µm as measured along the x-axis aligned perpendicular to the planar contact interface between the sapphire window and the titanium flange, the micro-scale diffusion bond has a diffusion bond width ranging from about 1 µm to about 200 µm extending along a y-axis into the sapphire window and the titanium washer, but not including undisturbed portions of the sapphire window and the titanium flange, and wherein the y-axis is aligned along the planar contact interface and intersects the x-axis at a right angle.

3. The optical sensor of claim 2, wherein the micro-scale diffusion bond has a diffusion bond width that is ≥ 45 µm extending along a y-axis that is aligned along the planar contact interface and intersects the x-axis at a right angle.

4. The optical sensor of any of claims 1 to 3, wherein the sapphire window:
(a) is optically transparent to electromagnetic (EM) radiation over a range of about 150 nm to about 6,000 nm;
(b) has a thickness extending to the window body fluid and device side surfaces that ranges from 100 µm to 4 mm; and/or
(c) has a material hardness of at least 9 on the Mohs hardness scale.

5. The optical sensor of any of claims 1 to 4, wherein the micro-scale diffusion bond connecting the sapphire window body fluid side surface to the flange device side surface is characterized as having been formed using a Gaussian-Bessel laser.

6. The optical sensor of any of claims 1 to 5, wherein the micro-scale diffusion bond connecting the sapphire window body fluid side surface to the flange device side surface has a strength that is as strong as at least one of the sapphire window and the titanium flange.

7. The optical sensor of any of claims 1 to 6, wherein the flange device side surface micro-scale diffusion bonded to the sapphire window body fluid side surface has a roughness (Ra) of less than 60 nm.

8. The optical sensor of any of claims 1 to 7, wherein the optical sensor is configured to detect optical signals from a human or animal body that pass through the sapphire window to impinge on the optical sensor of the PCB assembly,
optionally wherein the PCB assembly is configured to transmit an electromagnetic signal that is related to the detected optical signals.

9. The optical sensor of any of claims 1 to 8, wherein the electrical energy power source is rechargeable power source comprising a carbon-based or Li₄Ti₅O₁₂-based anode and a cathode selected from LiCoO₂ or lithium nickel manganese cobalt oxide (LiNiₐMn_{b}Co_{1-a-b}O₂).

10. The optical sensor of any of claims 1 to 9, further comprising a charging coil housed inside the device housing, wherein the charging circuit is connected to a charging circuit which is configured to convert RF or inductive energy received from the charging coil into a direct current voltage to charge the electrical energy power source that is configured to power the PCB assembly including the optical sensor.

11. The optical sensor of any of claims 1 to 10, wherein the housing sidewall comprises a first clamshell having a first clamshell rim and a second clamshell having a second clamshell rim, and wherein the first and second clamshell rims are welded to each other to form the open-ended container having the perimeter edge.

12. An optical sensor, comprising:
a) an open-ended container comprising a housing sidewall extending to an open perimeter;
b) a lid secured to the open perimeter of the housing sidewall to form a device housing defining an enclosed space, wherein the housing sidewall is comprised of titanium and has at least one housing opening communicating with the enclosed space;
c) a sapphire window comprising opposed planar window body fluid and device side surfaces, wherein a micro-scale diffusion bond having a thickness that is greater than 1 µm connects the sapphire window body fluid side surface to a planar inner surface of the housing sidewall aligned with the at least one housing opening so that the sapphire window diffusion bonded to the housing sidewall hermetically closes the at least one housing opening;
d) a printed circuit board (PCB) assembly housed inside the enclosed space of the device housing, the PCB assembly comprising a printed circuit board supporting at least an optical sensor, wherein the optical sensor is aligned with the sapphire window and the housing opening; and
e) an electrical energy power source that is configured to power the PCB assembly including the optical sensor.

13. The optical sensor of claim 12, wherein the micro-scale diffusion bond has a diffusion bond thickness that is ≥ 4 µm as measured along an x-axis aligned perpendicular to a planar contact interface between the sapphire window and the titanium flange, and wherein the diffusion bond thickness includes the combined depth of the diffusion bond extending from the planar contact interface along the x-axis into the sapphire window and the titanium washer, but not including undisturbed portions of the sapphire window and the titanium washer,
optionally wherein in addition to the micro-scale diffusion bond having the diffusion bond thickness that is ≥ 4 µm as measured along the x-axis aligned perpendicular to the planar contact interface between the sapphire window and the titanium flange, the micro-scale diffusion bond has a diffusion bond width ranging from about 1 µm to about 200 µm extending along a y-axis into the sapphire window and the titanium washer, but not including undisturbed portions of the sapphire window and the titanium flange, and wherein the y-axis is aligned along the planar contact interface and intersects the x-axis at a right angle.

14. The optical sensor of claim 12 or claim 13, wherein the sapphire window comprises at least one of:
a) optically transparency to electromagnetic (EM) radiation over a range of about 150 nm to about 6,000 nm;
b) a thickness extending to the window body fluid and device side surfaces that ranges from 100 µm to 4 mm; and
c) a material hardness of at least 9 on the Mohs hardness scale.

15. An assembly, comprising:
a) a titanium sidewall comprising opposed inner and outer sidewall surfaces, wherein at least one sidewall opening extends through the sidewall to the inner and outer sidewall surfaces;
b) a titanium flange comprising opposed flange first and second side surfaces surrounding a flange opening, wherein at least the flange first side surface is planar;
d) a sapphire window comprising opposed planar window first and second side surfaces, wherein a micro-scale diffusion bond having a diffusion bond thickness that is ≥ 4 µm connects the sapphire window second side surface to the flange first side surface aligned with the flange opening, and wherein a weld connects the flange second side surface to the inner surface of the sidewall aligned with the at least one sidewall opening so that the sapphire window diffusion bonded to the titanium flange in turn connected to the housing sidewall hermetically closes the at least one sidewall opening.
